# EUROPÄISCHE PATENTSCHRIFT

(11) **EP 1 480 985 B1**
(45) Veröffentlichungstag und Bekanntmachung des Hinweises auf die Patenterteilung: **09.03.2005**
(21) Anmeldenummer: 04700011.2
(22) Anmeldetag: 13.02.2004
(51) Int. Cl.: C07D 495/04, A61K 31/435

(54) **SALZ DER BENZOLSULFONSÄURE MIT CLOPIDOGREL UND DESSEN VERWENDUNG ZUR HERSTELLUNG PHARMAZEUTISCHER FORMULIERUNGEN**
SALT OF BENZOSULFONIC ACID CONTAINING CLOPIDOGREL AND USE THEREOF FOR THE PRODUCTION OF PHARMACEUTICAL FORMULATIONS
SEL DE L'ACIDE BENZENESULFONIQUE COMPRENANT DU CLOPIDOGREL ET SON UTILISATION POUR PRODUIRE DES FORMULATIONS PHARMACEUTIQUES

(30) Priorität: 13.02.2003 DE 10305984
(43) Veröffentlichungstag der Anmeldung: 01.12.2004
(73) Patentinhaber: Helm AG, 20097 Hamburg (DE)
(72) Erfinder: DOSER, Karlheinz, 22455 Hamburg (DE); GLÄNZER, Klaus, 22337 Hamburg (DE)
(74) Vertreter: Teipel, Stephan, Dr.
(86) Internationale Anmeldenummer: PCT/EP2004/001369
(87) Internationale Veröffentlichungsnummer: WO 2004/072084

(56) Entgegenhaltungen:
- US-A- 4 847 265

## Beschreibung

Die vorliegende Erfindung betrifft das Salz der Benzolsulfonsäure mit Clopidogrel, ein Verfahren zu dessen Herstellung sowie dessen Verwendung zur Herstellung pharmazeutischer Formulierungen. Die vorliegende Erfindung umfasst ferner Wirkstoffpartikel mit Clopidogrel-besylat.

Clopidogrel (5-Methyl-α-(4,5,6,7-tetrahydro[2,3-c]thienopyridyl)(2-chlorphenyl)acetat) ist als Wirkstoff aus EP-A-0 099 802 bekannt. Clopidogrel wirkt als Plättchenaggregationshemmer und kann daher beispielsweise zur Prävention thromboembolischer Ereignisse, wie z.B. Schlaganfall oder Myokardinfarkt, eingesetzt werden.

Die EP-A-0 281 459 schlägt vor, in pharmazeutischen Formulierungen anorganische Salze des (S)-(+)-Clopidogrels einzusetzen, insbesondere (S)-(+)-C)opidogrel-Hydrogensulfat. Dieses Dokument offenbart auch organische Salze des Clopidogrels, diese werden jedoch als amorph und/oder hygroskopisch sowie schwer zu reinigen beschrieben.

Das in pharmazeutischen Formulierungen eingesetzte (S)-(+)-Clopidogrel-Hydrogensulfat weist den Nachteil auf, dass zu seiner Herstellung konzentrierte Schwefelsäure benötigt wird und dass entsprechende Produkte wegen des aziden Protons stark sauer reagieren. Diese sauren Eigenschaften beeinflussen die Kompatibilität mit vielen pharmazeutischen Hilfsstoffen und damit die Stabilität entsprechender Arzneiformen negativ. Es besteht daher ein Bedürfnis nach stabilen, leicht zu reinigenden und leicht mit verschiedenen pharmazeutischen Hilfsstoffen, wie Arzneimittelträgern und -zusatzstoffen, verarbeitbaren Formen von Clopidogrel.

Eine Aufgabe der vorliegenden Erfindung besteht somit darin, Clopidogrel in einer Form zur Verfügung zu stellen, die leicht zu reinigen, stabil und auch in industriellem Maßstab leicht zu verarbeiten ist. Außerdem sollen Wechselwirkungen mit üblichen Arzneimittelträgern, -zusatzstoffen und -verarbeitungshilfsstoffen möglichst vermieden werden.

Es wurde nun überraschend gefunden, dass sich entgegen der Offenbarung der EP-A-0 281 459 das Salz der Benzolsulfonsäure mit Clopidogrel unter bestimmten Bedingungen zur Herstellung von pharmazeutischen Formulierungen eignet.

Die vorliegende Erfindung betrifft somit das Salz der Benzolsulfonsäure mit Clopidogrel, das zumindest teilweise in kristalliner Form vorliegt. Die vorliegende Erfindung betrifft außerdem das Salz der Benzolsulfonsäure mit Clopidogrel, herstellbar durch Fällen des Salzes aus einer Clopidogrel-Lösung, wobei das Lösungsmittel Toluol und/oder Dioxan umfasst.

Als Clopidogrel kann erfindungsgemäß ein racemisches Gemisch der beiden Clopidogrel-Isomere eingesetzt werden. Alternativ können die reinen Isomere verwendet werden, wobei das (S)-(+)-Clopidogrel-Isomer bevorzugt ist.

Erfindungsgemäß wurde überraschend gefunden, dass es entgegen der Lehre der EP-A-0 281 459 möglich ist, das Salz der Benzolsulfonsäure mit Clopidogrel in pharmazeutischen Formulierungen und insbesondere in oral zu verabreichenden pharmazeutischen Formulierungen einzuarbeiten. Die Erfindung umfasst somit auch die Verwendung des Salzes der Benzolsulfonsäure mit Clopidogrel zur Herstellung einer pharmazeutischen Formulierung sowie pharmazeutische Formulierungen, die ein solches Salz enthalten.

Das erfindungsgemäße Salz ist zumindest teilweise, vorzugsweise vollständig kristallin. In dieser Form lässt sich das Salz leichter reinigen als in den in der EP-A-0 281 459 offenbarten amorphen Formen. Außerdem lässt sich kristallines Salz leichter zu pharmazeutischen Formulierungen weiterverarbeiten.

Erfindungsgemäß wurde femer gefunden, dass sich die gewünschten und insbesondere kristallinen Salze der Benzolsulfonsäure mit Clopidogrel einfach und in einer für die Weiterverarbeitung zu einer pharmazeutischen Formulierung günstigen Form durch Fällen des Salzes aus einer Lösung von Clopidogrel herstellen lassen, wenn das Lösungsmittel Toluol und/oder Dioxan umfasst. Bevorzugt können Mischungen aus Toluol und Aceton oder Dioxan und Ethylacetat eingesetzt werden.

Beispielsweise kann die Clopidogrel-Base in Toluol gelöst und das gewünschte Salz durch Zugabe einer Benzolsulfonsäurelösung in Aceton ausgefällt werden. In einer anderen Ausführungsform können sowohl die Clopidogrel-Base als auch die Benzolsulfonsäure in Dioxan gelöst, vermischt und das gewünschte Salz durch Zugabe von Ethylacetat ausgefällt werden.

Nach dem vorstehend beschriebenen Verfahren lässt sich das Salz von Benzolsulfonsäure mit Clopidogrel in guter Ausbeute und Reinheit erhalten, so dass sich dieses Salz, insbesondere wenn es in kristalliner Form vorliegt, besonders zur Herstellung von pharmazeutischen Formulierungen eignet.

Es wurde außerdem gefunden, dass das Salz der Benzolsulfonsäure mit Clopidogrel dann besonders vorteilhafte Eigenschaften beispielsweise in Bezug auf seine Kristallinität aufweist, wenn es Lösungsmittelmoleküle enthält. Die in das Salz als Solvat eingelagerten Lösungsmittelmoleküle stammen aus der Lösung, aus der das Salz ausgefällt worden ist. Vorzugsweise enthält das Salz Toluol oder Dioxan.

Das aus Toluol ausgefällte Salz der Benzolsulfonsäure mit Clopidogrel enthält Toluolmoleküle. Die 10 intensitätsstärksten Peaks des Röntgenpulverspektrums dieses Salzes weisen die folgenden 2Θ-Werte auf:

| relative Intensität | 2Θ |
|---|---|
| 99,11 | 10,80 |
| 100,00 | 12,08 |
| 96,77 | 16,09 |
| 62,57 | 16,66 |
| 84,58 | 20,22 |
| 93,53 | 21,50 |
| 66,00 | 22,56 |
| 78,33 | 22,91 |
| 81,82 | 23,45 |
| 56,15 | 24,92 |

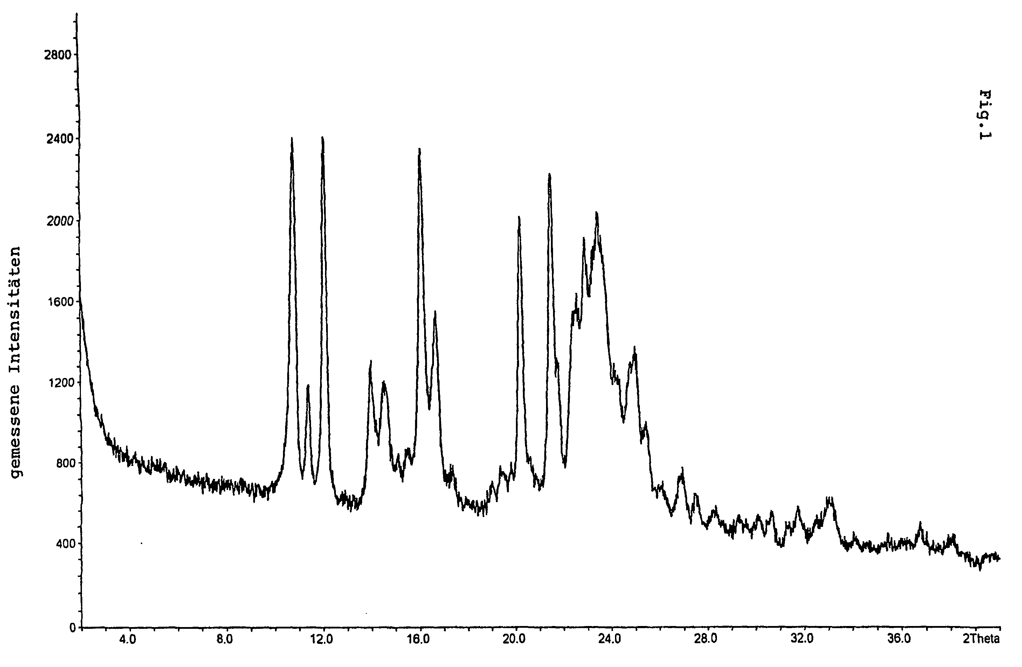

Das Röntgenpulverspektrum, das mit einem STOE STADI P Transmissionsdiffraktometer unter Verwendung von Kupfer-Kα-Strahlung aufgenommen wurde, ist in anliegender Figur 1 dargestellt.

Das aus Dioxan ausgefällte Salz enthält Dioxanmoleküle. Die 10 intensitätsstärksten Peaks des Röntgenpulverspektrums dieses Salzes weisen die folgenden 2⊖-Werte auf:

| relative Intensität | 2Θ |
|---|---|
| 51,66 | 10,78 |
| 54,15 | 10,87 |
| 90,13 | 12,13 |
| 50,83 | 14,34 |
| 50,27 | 16,43 |
| 76,03 | 21,57 |
| 81,19 | 22,87 |
| 100,00 | 23,06 |
| 54,18 | 23,72 |
| 54,05 | 25,17 |

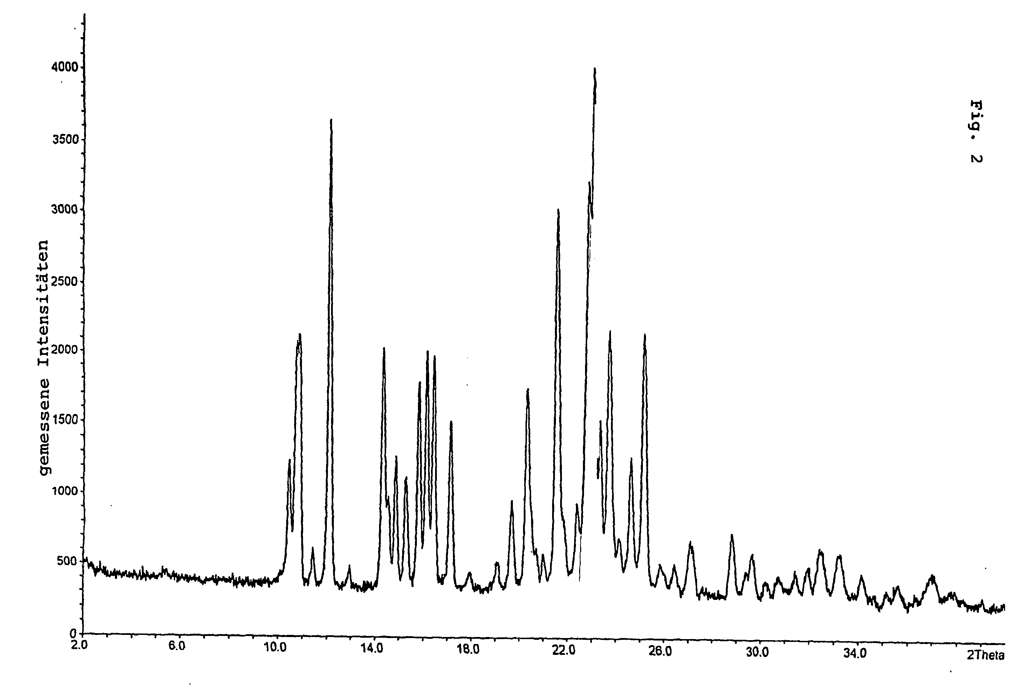

Das wie vorstehend beschrieben gemessene Röntgenpulverspektrum dieses Salzes ist in anliegender Figur 2 wiedergegeben.

Darüber hinaus wurde gefunden, dass das Salz der Benzolsulfonsäure mit Clopidogrel im Vergleich mit anderen Clopidogrel-Salzen in besonders hoher Reinheit gewonnen wird. Beim Auskristallisieren aus Dioxan wird beispielsweise ein Salz mit nur 0,085% Verunreinigungen (nach HPLC) erhalten. Daher eignet sich das erfindungsgemäße Salz für die Herstellung von reinem Clopidogrel. Die vorliegende Erfindung betrifft daher auch ein Verfahren zur Reinigung von Clopidogrel, wobei verunreinigtes Clopidogrel oder ein Salz davon, gegebenenfalls nach Freisetzung der Clopidogrel-Base, in das Salz der Benzolsulfonsäure mit Clopidogrel überführt wird und, falls gewünscht, anschließend aus dem isolierten Salz der Benzolsulfonsäure die Clopidogrel-Base freigesetzt und/oder in ein anderes Salz überführt wird.

Ein weiterer Aspekt der vorliegenden Erfindung besteht darin, das Salz der Benzolsulfonsäure mit Clopidogrel in einer einfach weiterverarbeitbaren Form zur Verfügung zu stellen. Dies wird erfindungsgemäß dadurch erreicht, dass das Satz auf ein festes Adsorbtionsmittel aufgebracht wird. Hierdurch werden Wirkstoffpartikel erhalten, die sich leicht schütten und dosieren lassen.

Als Adsorbtionsmittel eignet sich jeder physiologisch und pharmazeutisch akzeptable, vorzugsweise partikuläre Feststoff, der in der Lage ist, das Salz der Benzolsulfonsäure mit Clopidogrel zu adsorbieren. Vorzugsweise ist der Feststoff ein rieselfähiges Pulver, das leicht zu oralen pharmazeutischen Formulierungen weiterverarbeitet werden kann.

Physiologisch und pharmazeutisch akzeptable Adsorbtionsmittel sind beispielsweise:
1. Natürliche oder aufbereitete Adsorbtionsmittel aus der Gruppe der Tonerden (Tonmaterialien) und sonstiger Erden und Mineralien, z.B. Attapulgite, Aluminium-Magnesium-Silikate (Carrisorb®, Getsorb®), Magnesium-Aluminium-Silikate (Pharmasorb®, Veegum®), Magnesiumsilikate (Talkum), Calciumsilikate, Bentonite, Kaolin, Magnesium-Trisilikate, Montmorillonite, Porzellanerden (Bolus), Sepiolite (Meerschaum)
2. Kieselgele, Kieselgur, Kieselsäuren
3. Kolloidale (hochdisperse) Kieselsäuren (hydrophobe oder hydrophile Aerosile®, Cab-o-sile®)
4. Cellulosen, modifizierte Cellulosen, fein- und mikrokristalline Cellulosen sowie Cellulosederivate, Celluloseacetat, Cellulosefettsäureester, Cellulosenitrate, Celluloseether (Carboxymethylcellulosen, Ethylcellulosen, Hydroxyethylcellulosen, Hydroxypropylcellulosen, Methylcellulosen, Methylethylcellulosen, Methylhydroxypropylcellulosen)
5. Zucker und Zuckerderivate (Mono- und Polysaccharide), Lactosen, Dextrane, Dextrose, Cyclodextrine
6. Native Mais-, Reis-, Tapioka-, Weizen-, Kartoffelstärken und deren Derivate, Dextrine, prägelatinisierte, ganz oder teilweise hydrolysierte Stärken
7. Feste Polyole, insbesondere Mannit oder Sorbit
8. Polyacrylate, Acrylsäurepolymerisate bzw. Copolymerisate
9. Phosphate, Sulfate, Carbonate, Gluconate, Oxide von Alkali- und Erdalkalimetallen sowie physiologisch akzeptablen Schwer- und Übergangsmetallen
10. Guar-Mehl, Guar-Gummi
11. Johannisbrotmehl (Carob-Mehl, Carob-Gummi)
12. Alginsäure, Alginate und Algenmehl
13. Tragant
14. Carbo vegetabilis (Kohle)
15. Pektine und Amylopektine
16. N-Vinylpyrrolidon-Polymere, wie z.B. Povidon oder Crospovidon.

Die Adsorbtionsmittel können einzeln oder in Mischung aus zwei oder mehr Adsorbtionsrnittein eingesetzt werden. Außerdem können die erfindungsgemäßen Wirkstoffpartikel neben dem Adsorbtionsmittel übliche pharmazeutische Hilfsstoffe beispielsweise zur Herstellung von Direkttablettiermischungen bzw. zur Herstellung von Granulaten zur Weiterverarbeitung zu Arzneimitteln umfassen. Alternativ können die erfindungsgemäßen Wirkstoffpartikel nach ihrer Herstellung mit entsprechenden Hilfsstoffen vermischt und dann zu pharmazeutischen Formulierungen weiterverarbeitet werden.

Besonders bevorzugte Adsorbtionsmittel sind Lactose (z. B. Lactopress®). Mannit (z. B. Mannogem®) und Cellulose (z. B. Celphere®), insbesondere Lactose. Ein Granulat auf Basis von pyrogen hergestelltem Siliciumdioxid wird, obwohl möglich, vorzugsweise nicht als Adsorbtionsmittel eingesetzt.

Zur Desorptionssteuerung können geeignete Netzmittel eingesetzt werden. Zur Stabilitätsverbesserung können beispielsweise Antioxidantien, wie z.B. Ascorbinsäure und deren Salze, zugefügt werden. Weitere geeignete Hilfsmittel sind Emulgatoren, Lösungsmittel und Lösungsvermittler.

Die erfindungsgemäßen Wirkstoffpartikel können beispielsweise aus einem Lösungsmittel gewonnen werden, in dem das Adsorbtionsmittel unlöslich oder schwer löslich und das Salz der Benzolsulfonsäure mit Clopidogrel löslich ist. Hierzu kann das Adsorbtionsmittel in dem Lösungsmittel suspendiert werden. Vor oder nach dem Suspendierungsschritt kann das Salz der Benzolsulfonsäure mit Clopidogrel in dem Lösungsmittel gelöst werden. Der Wirkstoff kann dabei entweder direkt oder als Lösung in demselben oder einem anderen Lösungsmittel zugegeben werden. Anschließend werden die Wirkstoffpartikel, die das Salz der Benzolsulfonsäure mit Clopidogrel auf dem Adsorbtionsmittel aufgebracht umfassen, aus dem Lösungsmittel beispielsweise durch Verdampfen des Lösungsmittels gewonnen.

Als Lösungsmittel eignen sich alle üblichen Lösungsmittel, in denen das gewählte Adsorbtionsmittel nicht löslich oder schwer löslich und das Salz der Benzolsulfonsäure mit Clopidogrel löslich ist. Beispielsweise können die vorstehend zur Herstellung des Salzes beschriebenen Lösungsmittel verwendet werden. Alternativ kann beispielsweise Diethylether oder Methyl-tert-butylether eingesetzt werden.

In einer alternativen Ausführungsform des erfindungsgemäßen Verfahrens zur Herstellung von Wirkstoffpartikeln führt man die letzte Stufe der Synthese von Clopidogrel in Gegenwart des Adsorbtionsmittels durch. Dadurch können die gewünschten Wirkstoffpartikel ohne einen isolierenden Zwischenschritt hergestellt werden. Ferner können beispielsweise Clopidogrel und Benzolsulfonsäure mit der Suspension des Adsorbtionsmittels vermischt werden. Dabei können das Clopidogrel und die Benzolsulfonsäure jeweils getrennt in einem Lösungsmittel aufgelöst und gleichzeitig oder nacheinander zu der Suspension zugegeben werden. Alternativ können das Clopidogrel und die Benzolsulfonsäure in reiner Form zu der Suspension zugegeben werden. Einzelne Bestandteile können auch separat vorgemischt und dann gemeinsam zu der Suspension zugegeben werden.

Das Gewichtsverhältnis von Adsorbtionsmittel zu darauf adsorbiertem Salz der Benzolsulfonsäure mit Clopidogrel ist für die vorliegende Erfindung nicht besonders wesentlich und kann vom Fachmann in Abhängigkeit von dem gewünschten Verwendungszweck frei gewählt werden. Bei einer Weiterverarbeitung zu oralen, pharmazeutischen Formulierungen sollte jedoch beachtet werden, dass genügend Clopidogrel auf dem Adsorbtionsmittel aufgebracht ist, damit die gewünschte Dosierung in der Einheitsdosisform erreicht wird. Beispielsweise kann das Gewichtsverhältnis von Salz der Benzolsulfonsäure mit Clopidogrel, bezogen auf die freie Clopidogrel-Base, zu Adsorbtionsmittel im Bereich von 2:1 bis 1:6 liegen, d.h. z.B. 1 Gewichtsteil Clopidogrel-Base pro 6 Gewichtsteile Adsorbtionsmittel, vorzugsweise im Bereich von 1:1 bis 1:3.

Die vorliegende Erfindung wird durch die nachfolgenden Beispiele näher erläutert, ohne sie auf diese einzuschränken.

In den Beispielen wurden die Röntgenpulverspektren mit einem STOE STADI P Transmissionsdiffraktometer mit Kupfer-Kα-Strahlung, die NMR-Daten mit einem Varian Unityplus 300-Gerät und die CHN-Daten mit einem Carlo Erba Analyzer 1106 aufgenommen.

### Beispiet 1

### Darstellung von Clopidogrel-Benzolsulfonat aus Aceton/Toluol

4,0 g (12,5 mmol) Clopidogrel-Base wurden in 30 ml Toluol gelöst und dazu 2,0 g (12,5 mmol) wasserfreie Benzolsulfonsäure in 10 ml Aceton gegeben. Nach einiger Zeit und Verreiben mit einem Glasstab wird das Produkt fest und läßt sich absaugen. Das Produkt wurde über Nacht getrocknet unter Vakuum am Pumpenstand im Exsiccator.

Ausbeute: 67 % Fp. 87° - 90° C

NMR (ppm)
2.35 (Toluol), 3.0 - 3.5 und 3.8 - 4.3 (4 H), 3.79 (3 H), 4.8 - 5.2 (1H), 5.69(1 H), 6.6 - 6.8 (1 H), 7.2 - 8.0 (12H)

Das Röntgenpulverspektrum dieses Salzes ist in Figur 1 wiedergegeben.

Beim weiteren Trocknen bis zur vollständigen Entfernung des Toluols aus dem Salz bricht die Kristallstruktur zusammen und man erhält amorphes Clopidogrel-Benzolsulfonat.

### Beispiel 2

### Darstellung von Clopidogrel-Benzolsulfonat aus Dioxan

109,2 g (339,7 mmol) Clopidogrel-Base, in 300 ml Dioxan gelöst, werden bei 10° C unter Rühren mit einer Lösung von 53,7 g (339,7 mmol) wasserfreier Benzolsulfonsäure in 100 ml Dioxan versetzt. Zu dieser Lösung gibt man 250 ml Ethylacetat und stellt diese Lösung in den Tisfkühlschrank über Nacht. Man lässt die Lösung auf Raumtemperatur erwärmen, saugt das Adsorbat ab und wäscht mit Ethylacetat nach. Das Produkt wird unter Vakuum bei Raumtemperatur 48 Std. getrocknet.

Ausbeute: 71 % Fp. 93° - 95° C

| Elementaranalyse | | | |
|---|---|---|---|
| Werte [%] | berechnet für Clopidogrel-Besylat *1/2 Dioxan | gefunden | |
| C | 55,01 | 55,28 | 55,03 |
| H | 5,00 | 5,12 | 4,99 |
| N | 2,67 | 2,62 | 2,53 |

NMR (ppm)
3.0 - 3.5 und 3.8 - 4.3 (4 H), 3.79 (3 H), 4.8 - 5.2 (1 H), 5,68 - 5,72 (1 H), 6.6 - 6.8 (1H), 7.2 - 8.0 (12H), 3,70 (4 H; ½ Dioxan)

Das Röntgenpulverspektrum dieses Salzes ist in Figur 2 wiedergegeben.

### Beispiel 3

### Stabilitätsuntersuchungen

3.1 Die Stressstabilität verschiedener Salze des Clopidogrels wurde unter mehreren Bedingungen untersucht. Als Salze wurden die bislang als am stabilsten bekannte Form II des Clopidogrel-Hydrogensulfats, Clopidogrel-Hydrochlorid (hergestellt gemäß EP 281 459), amorphes Clopidogrel-Benzolsulfonat und kristallines Clopidogrel-Benzolsulfonat (aus vorstehendem Beispiel 2) eingesetzt. Folgende Versuche wurden durchgeführt:

### Stabilität unter sauren Bedingungen

50 mg des jeweiligen Salzes werden in einem volumetrischen Kolben (100 ml) eingewogen und mit 2 ml 1 N HCl versetzt. Anschließend wird der Kolben entweder 5 h bei Raumtemperatur oder 5 h bei 80° C aufbewahrt. Nach dem Ende des jeweiligen Experiments und gegebenenfalls Abkühlen auf Raumtemperatur werden 2 ml 1N NaOH zugegeben und es wird bis auf 100 ml mit der mobilen Phase aufgefüllt.

Das Ergebnis wird mittels HPLC bestimmt.

### Stabilität unter basischen Bedingungen

50 mg des jeweiligen Salzes werden in einem volumetrischen Kolben (100 ml) eingewogen und mit 2 ml 1 N NaOH versetzt. Anschließend wird der Kolben entweder 5 h bei Raumtemperatur oder 5 h bei 80° C aufbewahrt. Nach dem Ende des jeweiligen Experiments und gegebenenfalls Abkühlen auf Raumtemperatur werden 2 ml 1N HCl zugegeben und es wird bis auf 100 ml mit der mobilen Phase aufgefüllt.

Das Ergebnis wird mittels HPLC bestimmt.

### Stabilität unter oxidativen Bedingungen

50 mg des jeweiligen Salzes werden in einem volumetrischen Kolben (100 ml) eingewogen und mit 2 ml 3% H₂O₂ versetzt. Anschließend wird der Kolben entweder 5 h bei Raumtemperatur oder 5 h bei 80° C aufbewahrt. Nach dem Ende des jeweiligen Experiments und gegebenenfalls Abkühlen auf Raumtemperatur wird bis auf 100 ml mit der mobilen Phase aufgefüllt.

Das Ergebnis wird mittels HPLC bestimmt.

### Stabilität unter neutralen Bedingungen

50 mg des jeweiligen Salzes werden in einem volumetrischen Kolben (100 ml) eingewogen und mit 2 ml Wasser versetzt. Anschließend wird der Kolben entweder 5 h bei Raumtemperatur oder 5 h bei 80° C aufbewahrt. Nach dem Ende des jeweiligen Experiments und gegebenenfalls Abkühlen auf Raumtemperatur wird bis auf 100 ml mit der mobilen Phase aufgefüllt.

Das Ergebnis wird mittels HPLC bestimmt.

### Stabilität unter Wärmeeinfluss

50 mg des jeweiligen Salzes werden in einem volumetrischen Kolben (100 ml) eingewogen und 20 h bei 80° C aufbewahrt. Nach dem Ende des jeweiligen Experiments und Abkühlen auf Raumtemperatur wird bis auf 100 ml mit der mobilen Phase aufgefüllt.

Das Ergebnis wird mittels HPLC bestimmt.

Die HPLC-Messungen erfolgten in allen Fällen unter folgenden Bedingungen mit UV-Detektion:

| | | |
|---|---|---|
| Säule | Hypersil BDS 5 µm, 250 * 4.6 mm | |
| Mobile Phase | Methanol | 650 ml |
| | 0,05 M 1-Octansuifonsäure -Na-Salz (mit Triethylamin und Phosphorsäure auf pH 2,5 eingestellt). | 350 ml |
| Fiussrate | 1 ml/min | |
| Säulentemperatur | Raumtemperatur | |
| Wellenlänge | 215 nm | |
| Injection volume | 20 µl | |
| Retentionszeit | ca 15 min | |

Die Ergebnisse dieser Untersuchungen sind in den folgenden Tabellen 1 - 4 zusammengefasst:

### Clopidogrel-Hydrogensulfat

**Tabelle 1:**

| **Bedingung** | **Raumtemperatur** | **80°C** |
|---|---|---|
| sauer | 0.32 % | 2.96 % |
| alkalisch | 0.32 % | 59.48 % |
| oxidierend | 0.33 % | 3.50 % |
| neutral | 0.40 % | 1.63 % |
| Wärme | --- | 0.31 % |

### Clopidogrel-Hydrochlorid

**Tabelle 2:**

| **Bedingung** | **Raumtemperatur** | **80°C** |
|---|---|---|
| sauer | 1.86 % | 3.31 % |
| alkalisch | 1.86 % | 72.89 % |
| oxidierend | 1.83 % | 4.16 % |
| neutral | 1.84 % | 4.33 % |
| Wärme | --- | 32.43% |

### Clopidogrel-Benzolsulfonat (amorph)

**Tabelle 3:**

| **Bedingung** | **Raumtemperatur** | **80°C** |
|---|---|---|
| sauer | 0.64 % | 2.36 % |
| alkalisch | 0.64 % | 25.04 % |
| oxidierend | 0.83 % | 2.94 % |
| neutral | 0.85 % | 3.01 % |
| Wärme | --- | 11.52 % |

### Clopidogrel-Benzolsulfonat (kristallin)

**Tabelle 4:**

| **Bedingung** | **Raumtemperatur** | **800°C** |
|---|---|---|
| sauer | 0.14 % | 2.76 % |
| alkalisch | 0.14 % | 28.05 % |
| oxidierend | 0.13 % | 3.98 % |
| neutral | 0.19 % | 4.18 % |
| Wärme | --- | 4.52 % |

Man erkennt, dass entgegen der Lehre des EP 281 459 das amorphe Clopidogrel-Benzolsulfonat im Vergleich zu den Hydrogensulfat- und Hydrochlorid-Salzen des Clopidogrels eine vergleichbare und insbesondere unter alkalischen Bedingungen wesentlich erhöhte Stabilität aufweist. Darüber hinaus ist die Stabilität der kristallinen Form des Clopidogrel-Benzolsulfonats gegenüber der amorphen Form dieses Salzes insbesondere bei der für die Lagerung von pharmazeutischen Produkten wichtigen Raumtemperatur weiter erhöht. Kristallines Clopidogrel-Benzolsulfonat ist sogar stabiler als das bislang als am stabilsten bekannte und in pharmazeutischen Formulierungen eingesetzte Clopidogrel-Hydrogensulfat.

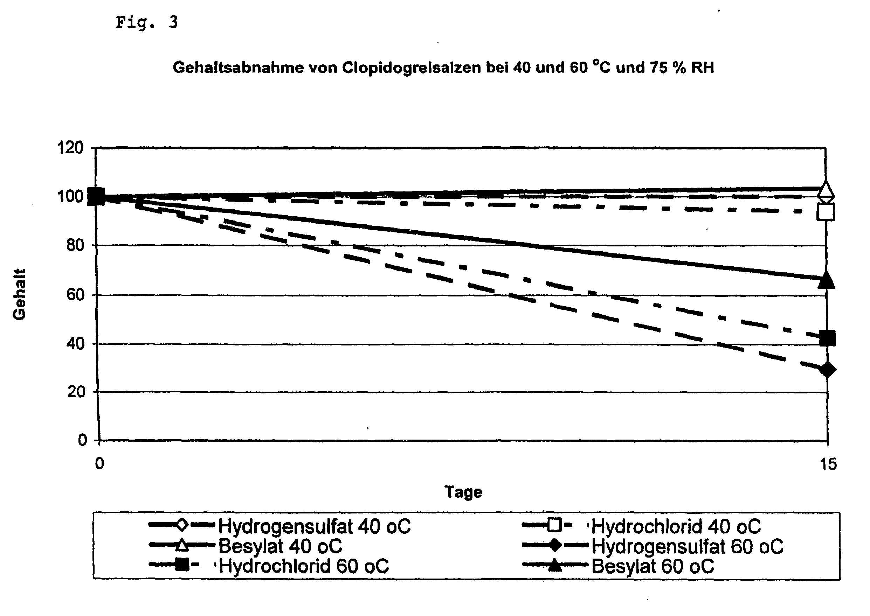

3.2 Darüber hinaus wurde die Gehaltsabnahme von Clopidogrel-Hydrogensulfat, -Hydrochlorid und -Besylat (kristallin) bei 40° und 60° C und 75% relativer Luftfeuchtigkeit über 15 Tage untersucht. Die Ergebnisse sind in der anliegenden Figur 3 wiedergegeben.

Man erkennt, dass sowohl bei 40° als auch bei 60° C das Besylatsalz (Clopidogrel-Benzolsulfonat) die beste Stabilität aufweist.

### Beispiel 4

### Adsorbat von (S)-(+)-Clopidogrel-Besylat an Calciumgluconat als Trägermaterial

Zu einer Lösung von 19,7 g (61,4 mmol) (S)-(+)-Clopidogrel in 300 ml wasserfreiem Diethylether wird bei 3° C langsam (ca. 30 min.) unter starkem Rühren eine Lösung von 11 g (69,5 mmol) wasserfreier Benzolsulfonsäure in 100 ml kalten, wasserfreien Diethylether getropft. Anschließend wird eine vorbereitete Aufschlämmung von 28 g Calciumgluconat in kaltem, wasserfreien Diethylether langsam zugegeben. Das nach Beendigung der Zugabe entstandene Adsorbat wird abgenutscht, mit eiskaltem, wasserfreien Diethylether gewaschen und anschließend getrocknet.

Man erhält ein weißes, rieselfähiges Pulver.

### Beispiel 5

### Adsorbat von (S)-(+)-Clopidogrel-Besylat an Kieselgel/Mannit als Trägermaterial

Bei einer Temperatur von 2° - 3° C werden 20 g (62,3 mmol) (S)-(+)-Clopidogrel und 11 g (69,5 mmol) wasserfreie Benzolsulfonsäure in 200 ml wasserfreiem Diethylether zur Reaktion gebracht. Anschließend wird eine Aufschlämmung von 2 g Kieselsäure und 20 g Mannit in 100 ml wasserfreiem Diethylether langsam zugegeben. Das entstandene Adsorbat wird in der Kälte abgenutscht, mit eiskaltem, wasserfreien Diethylether gewaschen und anschließend getrocknet.

Man erhält 39 g eines weißen, rieselfähigen Pulvers.

### Beispiel 6

Es wurden zwei verschiedene Verfahren zur Herstellung von Adsorbaten der Clopidogrel-Salze angewandt. Im ersten Verfahren wird das Salz in einem geeigneten Lösungsmittel gelöst und in dieser Lösung wird das Adsorbtionsmittel suspendiert.

In einer zweiten Versuchsreihe wurde die Clopidogrel-Base in einem geeigneten Lösungsmittel gelöst, das Adsorbtionsmittel wurde zugegeben und das Salz auf das Trägermaterial ausgefällt.

In allen Versuchen wurden jeweils Lactose (Lactopress®), Mannit (Mannogem®) und Cellulose (Celphere®) als Adsorbtionsmittel eingesetzt.

Folgende Versuche wurden durchgeführt:

### Clopidogrel-Besylat-Adsorbate mit Isolierung des Salzes

Es werden 1,5 g (3,1 mmol) Clopidogrel-Besylat in 20 ml Aceton gelöst und 1,5 g Adsorbtionsmittel zugegeben. Das Lösungsmittel wird abgezogen, der Rückstand kurz in MTB-Ether aufgeschlämmt und anschließend im Vakuum getrocknet.

### Clopidogrel-Besylat-Adsorbate ohne vorherige Isolierung der Salze

### 1. Lösungsmittel Diethylether

4,018 g (12,5 mmol) Clopidogrel-Base werden in 20 ml Diethylether gelöst. 6 g Adsorbtionsmittel und 1,977 g (12,5 mmol) Benzolsulfonsäure werden in 20 ml Ether zugegeben. Das Festprodukt wird abgesaugt, mit Ether gewaschen und im Vakuum getrocknet.

### 2. Lösungsmittel Methyl-tert-butylether (MTB-Ether)

4,018 g (12,5 mmol) Clopidogrel-Base werden in 40 ml MTB-Ether gelöst. 6 g Adsorbtionsmittel und 1,977 g (12,5 mmol) Benzolsulfonsäure werden in 50 ml MTB-Ether zugegeben. Das Festprodukt wird abgesaugt, mit MTB-Ether gewaschen und im Vakuum getrocknet.

### Beispiel 7

Die Stabilität der gemäß Beispiel 6 erhaltenen Adsorbate wurde untersucht. Die Adsorbate bleiben bei Raumtemperatur pulverig und verändern ihre Farbe über mehr als zwei Monate nicht.

Die Abnahme des Wirkstoffgehalts bei einer Lagerung über 15 Tage bei 40° bzw. 60° C und 75% relativer Luftfeuchtigkeit wurde gemessen. Die Ergebnisse sind in der nachfolgenden Tabelle 5 [Gehalt nach 15 Tagen (Anfangswert normiert auf 100%)] zusammengefasst.

**Tabelle 5**

| **Clopidogrel-Besylat** | **40°C** | **60°C** |
|---|---|---|
| Reines Salz | 103,32 | 66,48 |
| Lactopress/Diethytether | 106,91 | 94,47 |
| Lactopress/MTB-Ether | 94,74 | 92,58 |

Man erkennt, dass die Adsorbate gegenüber dem freien Salz bei erhöhter Temperatur eine größere Stabilität aufweisen.

### Beispiel 8

Gemäß Beispiel 6 hergestellte Adsorbate können direkt zu Tabletten verpresst werden. Dies wird durch die nachfolgenden Beispieiformulierungen verdeutlicht. Die verwendeten Mengen der in den nachfolgenden Beispielen angegebenen weiteren Hilfsstoffe sind dem Fachmann aufgrund seines Basiswissens bekannt und können Standardwerken zur Formulierung von Tabletten, wie z. B. Ritschel et al. "Die Tablette", Editio Cantor - Aulendorf, 2. Aufl., 2002, entnommen werden.

### a) Clopidogrel-Besylat-Mikrokristalline Cellulose-Adsorbat

Aus dem Adsorbat wurden Clopidogrel-Tabletten mit einer Gesamtmasse von 275 mg mittels Direktverpressung nach folgender Zusammensetzung hergestellt:
■ Clopidogrel-Besylat -Mikrokristalline Cellulose-Adsorbat (entspricht 75 mg Clopidogrel Base) 219,54 mg
■ Hilfsstoffe (Gleitmittel, Füllstoffe, Zerfallsbeschleuniger, Fließregulator, Netzmittel) ad 275 mg

Eigenschaften der pressfertigen Mischung und der Tabletten:
■ Kompressibilität und Fließfähigkeit: befriedigend bis gut
■ Mittlere Härte: 101 N
■ Abrieb: 0,11 %
■ Zerfallzeit: 65 Sek.
■ Freisetzung: 100% nach 30 Min.

Die so erhaltenen Tabletten können auch mit einem Überzug wie z. B. einem enterischen Überzug oder einem Überzug zur Geschmacksmaskierung versehen werden.

### b) Clopidogrel-Besylat Mannit Adsorbat

Aus dem Adsorbat wurden Clopidogrel-Tabletten mit einer Gesamtmasse von 275 mg mittels Direktverpressung nach folgender Zusammensetzung hergestellt:
■ Clopidogrel-Besylat-Mannit-Adsorbat (entspricht 75 mg Clopidogrel Base) 219,54 mg
■ Hilfsstoffe (Gleitmittel, Füllstoffe, Zerfallsbeschleuniger, Fließregulator, Netzmittel) ad 275 mg

Eigenschaften der pressfertigen Mischung und der Tabletten:
■ Kompressibilität und Fließfähigkeit: befriedigend bis gut
■ Mittlere Härte: 106 N
■ Abrieb: 0,15%
■ Zerfallzeit: 62 Sek.
■ Freisetzung: 100% nach 30 Min.

Die so erhaltenen Tabletten können mit einem Überzug wie z. B. einem enterischen Überzug oder einem Überzug zur Geschmacksmaskierung versehen werden.

### c) Clopidogrel-Besylat-Lactose-Adsorbat

Aus dem Adsorbat wurden Clopidogrel-Tabletten mit einer Gesamtmasse von 275 mg mittels Direktverpressung nach folgender Zusammensetzung hergestellt:
■ Clopidogrel-Besylat-Lactose-Adsorbat (entspricht 75 mg Clopidogrel Base) 219,54 mg
■ Hilfsstoffe (Gleitmittel, Füllstoffe, Zerfallsbeschleuniger, Fließregulator, Netzmittel) ad 275 mg

Eigenschaften der pressfertigen Mischung und der Tabletten:
■ Kompressibilität und Fließfähigkeit: befriedigend bis gut
■ Mittlere Härte: 96 N
■ Abrieb: 0,21%
■ Zerfallzeit: 76 Sek.
■ Freisetzung: 100% nach 30 Min.

Die so erhaltenen Tabletten können mit einem Überzug wie z. B. einem enterischen Überzug oder einem Überzug zur Geschmacksmaskierung versehen werden.

## Patentansprüche

1. Salz der Benzolsulfonsäure mit Clopidogrel, das zumindest teilweise in kristalliner Form vorliegt.

2. Salz der Benzolsulfonsäure mit Clopidogrel, herstellbar durch Fällen des Salzes aus einer Clopidogrel-Lösung, wobei das Lösungsmittel Toluol und/oder Dioxan umfasst.

3. Salz nach Anspruch 2, das zumindest teilweise in kristalliner Form vorliegt.

4. Salz nach einem der vorhergehenden Ansprüche, das Lösungsmittelmoleküle enthält.

5. Salz nach Anspruch 4, wobei das Lösungsmittel ausgewählt ist aus Toluol und Dioxan.

6. Salz nach Anspruch 5, das in kristalliner Form vorliegt und Toluol enthält, wobei die 10 intensitätsstärksten Peaks des Röntgenpulverspektrums dieses Salzes die folgenden 2Θ-Werte aufweisen:
| relative Intensität | 2Θ |
|---|---|
| 99,11 | 10,80 |
| 100,00 | 12,08 |
| 96,77 | 16,09 |
| 62,57 | 16,66 |
| 84,58 | 20,22 |
| 93,53 | 21,50 |
| 66,00 | 22,56 |
| 78,33 | 22,91 |
| 81,82 | 23,45 |
| 56,15 | 24,92 |

7. Salz nach Anspruch 6, das das in Figur 1 dargestellte Röntgenpulverspektrum aufweist.

8. Salz nach Anspruch 5, das in kristalliner Form vorliegt und Dioxan enthält, wobei die 10 intensitätsstärksten Peaks des Röntgenpulverspektrums dieses Salzes die folgenden 2Θ-Werte aufweisen:
| relative Intensität | 2Θ |
|---|---|
| 51,66 | 10,78 |
| 54,15 | 10,87 |
| 90,13 | 12,13 |
| 50,83 | 14,34 |
| 50,27 | 16,43 |
| 76,03 | 21,57 |
| 81,19 | 22,87 |
| 100,00 | 23,06 |
| 54,18 | 23,72 |
| 54,05 | 25,17 |

9. Salz nach Anspruch 8, das das in Figur 2 dargestellte Röntgenpulverspektrum aufweist.

10. Verfahren zur Herstellung eines Salzes nach einem der Ansprüche 1 - 9, wobei das Salz aus einer Lösung des Clopidogrels ausgefällt wird und das Lösungsmittel Toluol und/oder Dioxan umfasst.

11. Verfahren zur Reinigung von Clopidogrel, wobei verunreinigtes Clopidogrel oder ein Salz davon, gegebenenfalls nach Freisetzung der Clopidogrel-Base, in das Salz der Benzolsulfonsäure mit Clopidogrel überführt wird und, falls gewünscht, anschließend aus dem isolierten Salz der Benzolsulfonsäure die Clopidogrel-Base freigesetzt und/oder in ein anderes Salz überführt wird.

12. Verwendung eines Salzes nach einem der Ansprüche 1 - 9 zur Herstellung einer pharmazeutischen Formulierung.

13. Pharmazeutische Formulierung, umfassend ein Salz nach einem der Ansprüche 1 - 9.

14. Wirkstoffpartikel, umfassend ein festes Adsorbtionsmittel und darauf adsorbiertes Salz der Benzolsuflonsäure mit Clopidogrel.

15. Verwendung von Wirkstoffpartikeln nach Anspruch 14 zur Herstellung einer pharmazeutischen Formulierung.

16. Pharmazeutische Formulierung, umfassend Wirkstoffpartikel nach Anspruch 14.

17. Verfahren zur Herstellung von Wirkstoffpartikeln wie in Anspruch 14 definiert, umfassend das Gewinnen der Wirkstoffpartikel aus einem Lösungsmittel, in dem das Adsorbtionsmittel unlöslich oder schwer löslich und das Salz löslich ist.

18. Verfahren nach Anspruch 17, umfassend das Suspendieren des Adsorbtionsmittels in dem Lösungsmittel, das Lösen des Salzes in dem Lösungsmittel und das Gewinnen der Wirkstoffpartikel.

19. Verfahren nach Anspruch 17 oder 18, wobei die Wirkstoffpartikel durch Verdampfen des Lösungsmittels gewonnen werden.

20. Verfahren nach einem der Ansprüche 17 - 19, wobei Clopidogrel und Benzolsulfonsäure mit der Suspension des Adsorbtionsmittels vermischt werden.

## Claims

1. A salt of benzenesulfonic acid with clopidogrel which is at least partly in crystalline form.

2. A salt of benzenesulfonic acid with clopidogrel which can be prepared by precipitating the salt from a clopidogrel solution, where the solvent comprises toluene and/or dioxane.

3. The salt as claimed in claim 2, which is at least partly in crystalline form.

4. The salt as claimed in any of the preceding claims, which comprises solvent molecules.

5. The salt as claimed in claim 4, where the solvent is selected from toluene and dioxane.

6. The salt as claimed in claim 5, which is in crystalline form and comprises toluene, where the 10 most intense peaks in the x-ray powder spectrum of this salt have the following 2Θ values:
| relative intensity | 2Θ |
|---|---|
| 99.11 | 10.80 |
| 100.0 | 12.08 |
| 96.77 | 16.09 |
| 62.57 | 16.66 |
| 84.58 | 20.22 |
| 93.53 | 21.50 |
| 66.00 | 22.56 |
| 78.33 | 22.91 |
| 81.82 | 23.45 |
| 56.15 | 24.92 |

7. The salt as claimed in claim 6, which has the x-ray powder spectrum depicted in Figure 1.

8. The salt as claimed in claim 5, which is in crystalline form and comprises dioxane, where the 10 most intense peaks in the x-ray powder spectrum of this salt have the following 2Θ values:
| relative intensity | 2Θ |
|---|---|
| 51.66 | 10.78 |
| 54.15 | 10.87 |
| 90.13 | 12.13 |
| 50.83 | 14.34 |
| 50.27 | 16.43 |
| 76.03 | 21.57 |
| 81.19 | 22.87 |
| 100.00 | 23.06 |
| 54.18 | 23.72 |
| 54.05 | 25.17 |

9. The salt as claimed in claim 8, which has the x-ray powder spectrum depicted in Figure 2.

10. A process for preparing a salt as claimed in any of claims 1-9, where the salt is precipitated from a solution of clopidogrel, and the solvent comprises toluene and/or dioxane.

11. A process for purifying clopidogrel, where impure clopidogrel or a salt thereof is, where appropriate after liberation of clopidogrel base, converted into the salt of benzenesulfonic acid with clopidogrel and, if desired, subsequently clopidogrel base is liberated from the isolated salt of benzenesulfonic acid and/or is converted into another salt.

12. The use of a salt as claimed in any of claims 1-9 for producing a pharmaceutical formulation.

13. A pharmaceutical formulation comprising a salt as claimed in any of claims 1-9.

14. Active ingredient particles comprising a solid adsorbent and, adsorbed thereon, salt of benzenesulfonic acid with clopidogrel.

15. The use of active ingredient particles as claimed in claim 14 for producing a pharmaceutical formulation.

16. A pharmaceutical formulation comprising active ingredient particles as claimed in claim 14.

17. A process for producing active ingredient particles as defined in claim 14, comprising the obtaining of the active ingredient particles from a solvent in which the adsorbent is insoluble or slightly soluble and the salt is soluble.

18. The process as claimed in claim 17, comprising the suspension of the adsorbent in the solvent, the dissolving of the salt in the solvent, and the obtaining of the active ingredient particles.

19. The process as claimed in claim 17 or 18, where the active ingredient particles are obtained by evaporating the solvent.

20. The process as claimed in any of claims 17-19, where clopidogrel and benzenesulfonic acid are mixed with the suspension of the adsorbent.

## Revendications

1. Sel de l'acide benzène-sulfonique avec du clopidogrel, se présentant au moins en partie sous forme cristalline.

2. Sel de l'acide benzène-sulfonique avec du clopidogrel, pouvant être préparé en précipitant le sel d'une solution de clopidogrel, dans laquelle le solvant contient du toluène et/ou du dioxane.

3. Sel selon la revendication 2, se présentant au moins en partie sous forme cristalline.

4. Sel selon l'une quelconque des revendications précédentes, contenant des molécules de solvant.

5. Sel selon la revendication 4, dans lequel le solvant choisi est le toluène et le dioxane.

6. Sel selon la revendication 5, se présentant sous forme cristalline et qui contient du toluène, dans lequel les 10 pics les plus intenses du spectre rayons X de la poudre de ce sel ont les valeurs 2θ suivantes :
| Intensité relative | 2θ |
|---|---|
| 99,11 | 10,80 |
| 100,0 | 12,08 |
| 96,77 | 16,09 |
| 62,57 | 16,66 |
| 84,58 | 20,22 |
| 93,53 | 21,50 |
| 66,00 | 22,56 |
| 78,33 | 22,91 |
| 81,82 | 23,45 |
| 56,15 | 24,92 |

7. Sel selon la revendication 6, dont le spectre de diffraction des rayons X de la poudre de ce sel est représenté sur la Figure 1.

8. Sel selon la revendication 5, se présentant sous forme cristalline et qui contient du dioxane, dans lequel les 10 pics les plus intenses du spectre de diffraction des rayons X de la poudre de ce sel ont les valeurs 2θ suivantes :
| Intensité relative | 2θ |
|---|---|
| 51,66 | 10,78 |
| 54,15 | 10,87 |
| 90,13 | 12,13 |
| 50,83 | 14,34 |
| 50,27 | 16,43 |
| 76,03 | 21,57 |
| 81,19 | 22,87 |
| 100,00 | 23,06 |
| 54,18 | 23,72 |
| 54,05 | 25,17 |

9. Sel selon la revendication 8, dont le spectre de diffraction des rayons X de la poudre est représenté sur la Figure 2.

10. Procédé de préparation d'un sel selon l'une quelconque des revendications 1 à 9, dans lequel le sel d'une solution de clopidogrel est précipité, et le solvant contient du toluène et/ou du dioxane.

11. Procédé de purification du clopidogrel, dans lequel du clopidogrel impur ou un sel correspondant est, le cas échéant après libération de la base clopidogrel, converti en sel de l'acide benzène-sulfonique avec le clopidogrel et, si souhaité, la base de clopidogrel est par la suite libérée du sel, isolée de l'acide benzène-sulfonique et/ou convertie en un autre sel.

12. Utilisation d'un sel selon l'une quelconque des revendications 1 à 9 pour la préparation d'une formulation pharmaceutique.

13. Formulation pharmaceutique contenant un sel selon l'une quelconque des revendications 1 à 9.

14. Particules de substance active contenant un adsorbant solide et sur ce un sel adsorbé de l'acide benzène-sulfonique avec le clopidogrel.

15. Utilisation de particules de substance active selon la revendication 14 pour la préparation d'une formulation pharmaceutique.

16. Formulation pharmaceutique contenant des particules de substance active selon la revendication 14.

17. Procédé de préparation de particules de substance active comme définies dans la revendication 14, comprenant l'obtention de particules de substance active à partir d'un solvant dans lequel l'adsorbant est insoluble ou légèrement soluble et le sel est soluble.

18. Procédé de préparation selon la revendication 17, comprenant la suspension de l'adsorbant dans le solvant, la dissolution du sel dans le solvant, et l'obtention de particules de substance active.

19. Procédé selon la revendication 17 ou 18, dans lequel les particules de substance active sont obtenues en évaporant le solvant.

20. Procédé selon l'une quelconque des revendications 17 à 19, dans lequel le clopidogrel et l'acide benzène-sulfonique sont mélangés à la suspension de l'adsorbant.
